# EUROPÄISCHE PATENTANMELDUNG

(11) **EP 4 218 547 A1**
(43) Veröffentlichungstag der Anmeldung: **02.08.2023**
(21) Anmeldenummer: 22153623.8
(22) Anmeldetag: 27.01.2022
(51) Int. Cl.: A61B 5/00, A61B 5/103

(54) **KARIESERFASSUNGVORRICHTUNG**

(71) Anmelder: Ivoclar Vivadent AG, 9494 Schaan (LI)
(72) Erfinder: RAMPF, Markus, 7212 Seewis Dorf (CH); TICHY, Raimund, 6800 Feldkirch (AT); REINHARDT, Jonas, 7206 Igis (CH); GROSSE-HONEBRINK, Alexander, 9320 Arbon (CH); NIEDRIG, Christian, 9464 Rüthi (CH); KUHN, Marvin, 6800 Feldkirch (AT)
(74) Vertreter: Baldus, Oliver

(57) **Zusammenfassung**

Die vorliegende Erfindung betrifft eine Karieserfassungsvorrichtung (100), mit fluoreszierenden Partikeln (101), die bei Kontakt mit einer Schwefelverbindung die Fluoreszenzeigenschaft verlieren.

## Beschreibung

Die vorliegende Erfindung betrifft eine Karieserfassungsvorrichtung, ein Karieserfassungssystem mit einer Karieserfassungsvorrichtung und mehrere Verfahren zum Herstellen einer Karieserfassungsvorrichtung.

Kariöse Stellen von Zähnen in einem Gebiss können auf unterschiedliche Weise erkannt werden, wie beispielsweise durch Röntgenuntersuchung, mit einer mechanischen Sonde, durch Transillumination oder durch Schmerzen beim Patienten.

Es ist die technische Aufgabe der vorliegenden Erfindung, eine Karieserfassungsvorrichtung bereitzustellen, mit der kariöse Stellen in einem Gebiss auf einfache Weise erfasst werden können, ohne dass hierbei zu einer Strahlenbelastung kommt.

Diese technische Aufgabe wird durch Gegenstände nach den unabhängigen Ansprüchen gelöst. Technisch vorteilhafte Ausführungsformen sind Gegenstand der abhängigen Ansprüche, der Beschreibung und der Zeichnungen.

Gemäß einem ersten Aspekt wird die technische Aufgabe durch eine Karieserfassungsvorrichtung gelöst, mit fluoreszierenden Partikeln, die bei Kontakt mit einer Schwefelverbindung die Fluoreszenzeigenschaft verlieren. Zur Erfassung von kariösen Stellen eines Gebisses werden Partikel verwendet, die ihre Fluoreszenzeigenschaft verändern, sobald diese in Kontakt mit Schwefelverbindungen geraten, die von den kariösen Stellen freigesetzt werden. Fluoreszenz ist die spontane Emission von Licht kurz nach der Anregung eines Materials durch Licht. Dabei sind die emittierten Photonen in der Regel energieärmer als die vorher absorbierten. Sind die Partikel in Kontakt mit den Schwefelverbindungen gekommen, verlieren diese ihre zuvor vorhandene Fluoreszenzeigenschaft. Bei späterer Bestrahlung mit Licht erscheinen diese Partikel dann dunkler, als wenn diese nicht mit den Schwefelverbindungen in Kontakt gekommen wären.

Dunkle (nicht-fluoreszierende) Stellen der Karieserfassungsvorrichtung zeigen daher eine vorhandene kariöse Läsion an, von der Schwefelverbindungen ausgehen.

Durch die Karieserfassungsvorrichtung wird beispielsweise der technische Vorteil erreicht, dass kariöse Stellen erkannt werden können, ohne dass hierzu Röntgenstrahlung verwendet werden muss und keine Strahlenbelastung auftritt. Zudem können auch kariöse Stellen lokalisiert werden, die von außen nicht sichtbar sind, beispielsweise wenn diese unter einer Krone liegen, da die Schwefelverbindungen ebenfalls die Karieserfassungsvorrichtung erreichen. Die Handhabung der Karieserfassungsvorrichtung ist einfach, so dass diese von einem Benutzer auch im privaten Bereich verwendet werden kann.

In einer technisch vorteilhaften Ausführungsform der Karieserfassungsvorrichtung umfasst die Schwefelverbindung Schwefelwasserstoff oder organische Schwefelverbindungen. Die organischen Schwefelverbindungen umfassen beispielsweise schwefelhaltige Proteine, Methanthiol, Methylmercaptan oder Dimethylsulfid. Dadurch wird beispielsweise der technische Vorteil erreicht, dass kariöse Stellen innerhalb des Gebisses zuverlässig erkannt werden können.

In einer weiteren technisch vorteilhaften Ausführungsform der Karieserfassungsvorrichtung umfassen die fluoreszierenden Partikel Zinksulfid oder Cadmiumtellurid. Dadurch wird beispielsweise der technische Vorteil erreicht, dass ein starker Fluoreszenzeffekt bei gleichzeitiger hoher Sensitivität erreicht wird und kariöse Stellen gut erkannt werden können. Ein Kontrast von fluoreszierenden und nicht fluoreszierenden Bereichen ist hoch und erfordert wenig Schwefelgase für die Zerstörung der Fluoreszenzeigenschaft.

In einer weiteren technisch vorteilhaften Ausführungsform der Karieserfassungsvorrichtung sind die fluoreszierenden Partikel durch Quantenpunkte gebildet. Ein Quantenpunkt ist eine nanoskopische Materialstruktur. Die Ladungsträger, wie beispielsweise Elektronen oder Löcher, in einem Quantenpunkt sind in ihrer Beweglichkeit in allen drei Raumrichtungen so weit eingeschränkt, dass ihre Energie nicht mehr kontinuierliche, sondern nur noch diskrete Werte annehmen kann. Dadurch wird beispielsweise der technische Vorteil erreicht, dass sich die Fluoreszenzeigenschaften gezielt einstellen lassen.

In einer weiteren technisch vorteilhaften Ausführungsform der Karieserfassungsvorrichtung weisen die Quantenpunkte eine vorgegebene Form und/oder vorgegebene Abmessung auf. Die Form, Größe oder die Anzahl von Elektronen in Quantenpunkten kann unterschiedlich vorgegeben sein. Dadurch wird beispielsweise der technische Vorteil erreicht, dass sich die elektronischen und optische Eigenschaften der Quantenpunkten gezielt steuern lassen. Typischerweise beträgt die atomare Größenordnung ca. 10⁴ Atome.

In einer weiteren technisch vorteilhaften Ausführungsform der Karieserfassungsvorrichtung sind die fluoreszierenden Partikel oder Quantenpunkte in einer vorgegebenen dreidimensionalen Struktur angeordnet. Beispielsweise können die fluoreszierenden Partikel oder Quantenpunkte mit gleichmäßigen Abständen in der Karieserfassungsvorrichtung oder in einer kubischen oder hexagonalen dreidimensionalen Struktur angeordnet sein. Dadurch wird beispielsweise der technische Vorteil erreicht, dass eine genauere Erfassung der Fluoreszenz oder des Fluoreszenz-Quenchings ermöglicht wird.

In einer weiteren technisch vorteilhaften Ausführungsform der Karieserfassungsvorrichtung sind die fluoreszierenden Partikel oder Quantenpunkte homogen oder in einer vorgegebenen Dichte angeordnet. Die Dichte der fluoreszierenden Partikel oder Quantenpunkte beträgt beispielsweise 0.01 - 1.0 Massenprozent, vorzugsweise 0.01 - 0.1 Massenprozent. Dadurch wird beispielsweise der technische Vorteil erreicht, dass sich aufgrund der Dichtereferenz genauere Messwerte in Bezug zu dem Ausmaß einer kariösen Läsion gewinnen lassen.

In einer weiteren technisch vorteilhaften Ausführungsform der Karieserfassungsvorrichtung sind die fluoreszierenden Partikel in einem ersten räumlichen Bereich der Karieserfassungsvorrichtung in einer ersten Dichte angeordnet und einem zweiten räumlichen Bereich der Karieserfassungsvorrichtung in einer zweiten Dichte angeordnet. Dadurch wird beispielsweise der technische Vorteil erreicht, dass in bestimmten Bereichen der Karieserfassungsvorrichtung die Sensitivität zur Karieserfassung erhöht wird.

In einer weiteren technisch vorteilhaften Ausführungsform der Karieserfassungsvorrichtung ist die Karieserfassungsvorrichtung eine Zahnbürste, eine Zahnschiene, eine Aufbissschiene, eine Aligner-Schiene, eine Kunststofffolie, eine Zahnseide, ein Kaugummi, eine Teilprothese, ein Lack, ein Zement, oder ein Bracket oder ein elektronisches Gerät. Dadurch wird beispielsweise der technische Vorteil erreicht, dass die Karieserfassung von Gegenständen durchgeführt werden kann, die bereits zu einem anderen Zweck im Dentalbereich eingesetzt werden.

Gemäß einem zweiten Aspekt wird die technische Aufgabe durch ein Karieserfassungssystem mit einer Karieserfassungsvorrichtung nach dem ersten Aspekt und einer Lichtquelle zum Emittieren von Strahlung mit einer Wellenlänge gelöst, die kleiner als 500 nm ist. Die Lichtquelle ist beispielsweise eine Leuchtdiode oder eine Laserdiode. Dadurch wird beispielsweise der technische Vorteil erreicht, dass kariöse Stellen direkt optisch erfasst werden können.

In einer technisch vorteilhaften Ausführungsform des Karieserfassungssystems umfasst das Karieserfassungssystem einen Lichtsensor zum Erfassen von Fluoreszenzlicht. Der Lichtsensor kann beispielsweise durch eine elektronische Kamera gebildet sein. Dadurch wird beispielsweise der technische Vorteil erreicht, dass die Intensität oder ein Bild des Fluoreszenzabnahme erfasst werden kann.

Gemäß einem dritten Aspekt wird die technische Aufgabe durch ein Verfahren zum Herstellen einer Karieserfassungsvorrichtung gelöst, mit den Schritten eines Herstellens der Karieserfassungsvorrichtung in einem Formgebungsverfahren; und eines Einbringens von fluoreszierenden Partikeln in die Karieserfassungsvorrichtung, die bei Kontakt mit einer Schwefelverbindung die Fluoreszenzeigenschaft verlieren. Dadurch werden die gleichen technischen Vorteile wie durch die Karieserfassungsvorrichtung nach dem ersten Aspekt erreicht.

In einer technisch vorteilhaften Ausführungsform des Verfahrens wird die Karieserfassungsvorrichtung in einem dreidimensionalen Druckverfahren hergestellt und die fluoreszierenden Partikel werden durch das Druckverfahren in die Karieserfassungsvorrichtung eingebracht. In dem Druckverfahren kann eine homogene Mischung aus dem Herstellungsmaterial und den Partikeln verwendet werden. Die Dichte an fluoreszierenden Partikeln ist homogen. Die Partikel können aber auch während dem dreidimensionalen Druckverfahren durch einen eigenen Kanal, wie beispielsweise eine Düse, an bestimmten Stellen der Karieserfassungsvorrichtung mittels des Druckverfahrens angeordnet werden. Dadurch wird beispielsweise der technische Vorteil erreicht, dass die Karieserfassungsvorrichtung schnell und effizient in einer vorgegebenen Form hergestellt werden kann.

In einer weiteren technisch vorteilhaften Ausführungsform des Verfahrens wird die Karieserfassungsvorrichtung durch Tiefziehen einer Folie hergestellt und die fluoreszierenden Partikel werden durch die Folie in die Karieserfassungsvorrichtung eingebracht. Die fluoreszierenden Partikel sind in der Folie eingearbeitet, bevor diese tiefgezogen wird. Dadurch wird beispielsweise der technische Vorteil erreicht, dass die Karieserfassungsvorrichtung auf einfache Weise hergestellt werden kann.

In einer weiteren technisch vorteilhaften Ausführungsform des Verfahrens wird die Karieserfassungsvorrichtung durch Herausfräsen aus einem Rohling hergestellt und die fluoreszierenden Partikel werden durch den Rohling in die Karieserfassungsvorrichtung eingebracht. Die fluoreszierenden Partikel sind in dem Rohling eingearbeitet, bevor dieser gefräst wird. Dadurch wird beispielsweise der technische Vorteil erreicht, dass aus einem Rohling mit einer bestimmten Dichte an fluoreszierenden Partikeln hergestellt werden kann.

In einer weiteren technisch vorteilhaften Ausführungsform des Verfahrens werden die fluoreszierenden Partikel durch Aufbringen eines Lackes eingebracht. Dadurch wird beispielsweise der technische Vorteil erreicht, dass der Lack nachträglich zur Karieserfassung auf die Karieserfassungsvorrichtung aufgebracht werden kann.

In einer weiteren technisch vorteilhaften Ausführungsform des Verfahrens umfasst dieses den Schritt eines Emittierens von Strahlung durch eine Lichtquelle mit einer Wellenlänge, die kleiner als 500 nm ist. Dadurch wird beispielsweise der technische Vorteil erreicht, dass das nach der Herstellung die Fluoreszenz der Karieserfassungsvorrichtung überprüft werden kann oder nach einem Einsatz der Karieserfassungsvorrichtung beim Patienten kariöse Stellen direkt optisch erfasst werden können.

Ausführungsbeispiele der Erfindung sind in den Zeichnungen dargestellt und werden im Folgenden näher beschrieben.

Es zeigen:
- Fig. 1: eine schematische Ansicht einer Bissschiene;
- Fig. 2: eine weitere schematische Ansicht einer Bissschiene;
- Fig. 3: eine schematische Ansicht eines elektronischen Dentalgeräts als Karieserfassungsvorrichtung; und
- Fig. 4: eine schematische Ansicht eines Karieserfassungssystems.

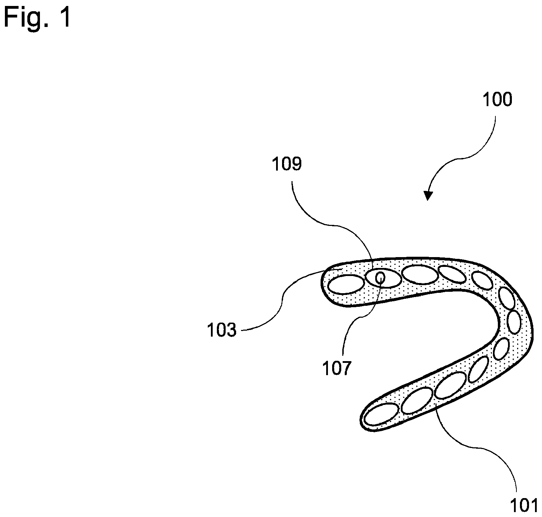

Fig. 1 zeigt eine schematische Ansicht einer Bissschiene 100 als Karieserfassungsvorrichtung. Die Aufgabe der Karieserfassungsvorrichtung besteht darin, kariöse aktive Läsionen eines Zahnes zu detektieren. Zu diesem Zweck werden Schwefelverbindungen verwendet, die von den kariösen aktiven Läsionen verursacht werden. Die Bissschiene umfasst beispielsweise fluoreszierende Partikel 101 aus Zinksulfid (ZnS) oder Cadmiumtellurid (CdTe).

Die fluoreszierenden Partikel können Quanten-Punkte sein. Es können fluoreszierende Partikel 101, beispielsweise Quanten-Punkte (QD - Quantum-Nanodots) basierend auf Zinksulfid, in die Bissschiene 100 eingearbeitet werden. Die Bissschiene 100 ist aus transparentem Material gebildet, das mehrere Stunden nahe bei der kariösen Stelle 107 eines Zahns 109 angeordnet ist.

Wenn die fluoreszierenden Partikel 101 in Kontakt mit den Schwefelverbindungen aus den Läsionen treten, verlieren diese aufgrund eines Fluoreszenz-Quenchings ihre Fluoreszenzeigenschaft genutzt. Die verursachten Schwefelverbindungen sind beispielsweise schwefelhaltige Proteine, Methanthiol, Methylmercaptan oder Dimethylsulfid.

Die Bissschiene 100 kann auch mit einem 3D-Drucker gedruckt werden oder aus einem aushärtendem Zweikomponenten-Material hergestellt werden. Bei einer Beaufschlagung mit einem Licht, das die fluoreszierenden Partikel 101 zum Leuchten bringt, leuchtet die ganze Bissschiene 100 zunächst homogen. Wird die Bissschiene 100 eine Zeitlang getragen, so dass die Partikel 101 mit den Schwefelverbindungen in Kontakt kommen, wird eine Fluoreszenzabnahme der fluoreszierenden Partikel 101 hervorgerufen. Dadurch nimmt an diesen Stellen der Bissschiene 100 die Leuchtkraft der Fluoreszenz ab oder geht vollständig verloren. Daher können bei anschließender Beleuchtung der Bissschiene 100 die kariösen Stellen 107 an den Zähnen 109 ausfindig gemacht werden. Die Bissschiene 100 kann zusätzlich zu den fluoreszierenden Partikeln 101 Fluoride umfassen, um Zähne während dem Tragen der Bissschiene 100 zu fluoridieren.

Die Bissschiene ist mit geringem Aufwand herzustellen. Es entsteht keine Strahlenbelastung bei der Lokalisation von kariösen Stellen 107. Die Bissschiene kann zu Hause oder nur zu Routineprüfungen verwendet werden. Zudem erfolgt eine Detektion der mikrobiologischen Belastung (aktive Läsion).

Die Karieserfassungsvorrichtung kann nicht nur durch eine Bissschiene, sondern auch durch eine Zahnbürste (Borsten), eine Zahnschiene, eine Aligner-Schiene, eine Kunststofffolie, eine Zahnseide, ein Kaugummi, eine Teilprothese, ein Lack, ein Zement, oder ein Bracket oder ein elektronisches Dentalgerät gebildet sein. All diese Gegenstände können die fluoreszierenden Partikel 101 umfassen, um kariöse Strukturen zu erkennen. Bei der Integration der fluoreszierende Partikel 101 in eine strukturierte Folie kann diese als Kariesdetektionsfolie verwendet werden.

Die fluoreszierenden Partikel 101 können an unterschiedlichen Stellen der Karieserfassungsvorrichtung in einer unterschiedlichen Konzentration oder Dichte vorgesehen sein, um eine semi-quantitative Auswertung zu ermöglichen.

Daneben können die fluoreszierenden Partikel 101 durch Quantenpunkte 103 gebildet sein, die verschiedene Spektren erzeugen (Anregung und Emission) und auf verschiedene Gase reagieren. Weiter ist es möglich, dass Quantenpunkte 103 derart ausgebildet sind, dass diese erst nach einer bestimmten Zeit auf die jeweilige Schwefelverbindung reagieren. Dadurch kann ein zeitlicher Verlauf einer Strukturveränderung des Zahnes 109 erfasst werden.

Die Quantenpunkte 103 sind fluoreszierende Nanopartikel bestimmter Halbleiterkristalle, deren elektronische Eigenschaften stark von der Partikelgröße abhängig sind, wie beispielsweise Zn-Cu-In-S oder ZnS. Je kleiner die Quantenpunkte 103, umso stärker ist die Fluoreszenzfarbe in Richtung blau verschoben. Durch Quantenpunkte 103 ist es möglich, eine Emission des gesamten Farbspektrums aus demselben Material mit verschiedenen Durchmessern zu erzeugen.

Da die Größe der Quantenpunkte 103 während ihrer Herstellung präzise eingestellt werden kann, lassen sich deren elektronische und Fluoreszenzeigenschaften genau steuern. Quantenpunkte 103 weisen aufgrund ihres Aufbaus aus anorganischem Material eine hohe Stabilität auf. Außerdem können CdTe-Quantenpunkte 103 mit Carboxylgruppen (-COOH) funktionalisiert sein, so dass diese wasserlöslich sind und eine kovalente Bindung an Proteine und andere Moleküle ermöglichen. Die Quantenpunkte 103 weisen beispielsweise eine kugelförmige, pyramidale, quaderförmige, Zylinderförmige oder tetraedrische Geometrie auf. Die Quantenpunkte 103 weisen eine Abmessung von 1 bis 50 nm auf.

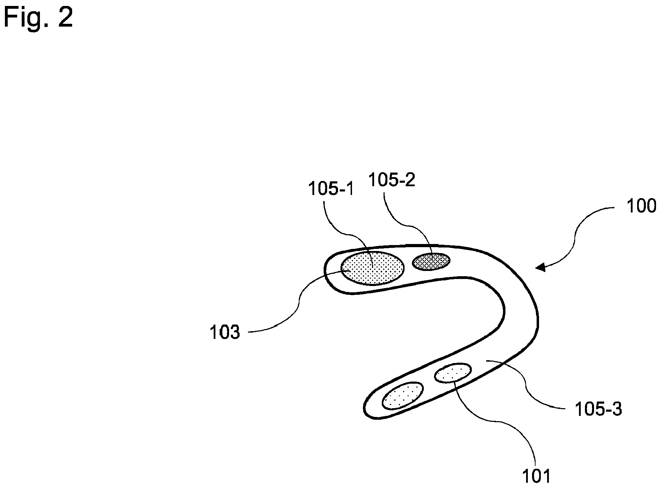

Fig. 2 zeigt eine weitere schematische Ansicht einer Bissschiene 100 als Karieserfassungsvorrichtung. In einem ersten räumlichen Bereich 105-1 der Bissschiene 100 sind die fluoreszierenden Partikel 101 in einer ersten Dichte angeordnet sind und in einem zweiten räumlichen Bereich 105-2 der Bissschiene 100 sind die fluoreszierenden Partikel 101 in einer zweiten Dichte angeordnet. Die erste Dichte und die zweite Dichte sind unterschiedlich zueinander. Die Quantenpunkte der fluoreszierenden Partikel 101 in dem ersten Bereich 105-1 der Bissschiene 100 können zudem eine andere Größe oder Form als die Quantenpunkte der fluoreszierenden Partikel 101 in dem zweiten Bereich 105-1 der Bissschiene 100 aufweisen. Dazwischen können auch räumliche Bereiche 105-3 der Karieserfassungsvorrichtung liegen, die ohne fluoreszierende Partikel 101 gebildet sind.

Auf diese Art und Weise gelingt es je nach räumlichem Bereich, die Sensitivität der Karieserfassungsvorrichtung zu erhöhen oder zu vermindern oder unterschiedles Fluoreszenzlicht auszusenden.

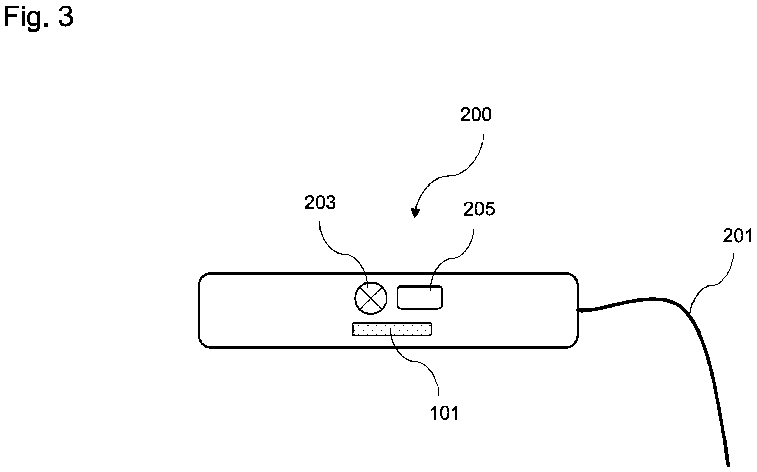

Fig. 3 zeigt eine schematische Ansicht eines elektronischen Dentalgeräts 200 als Karieserfassungsvorrichtung. Das elektronische Dentalgerät 200, wie beispielsweise ein tragbarer Stift (Pen), saugt die flüchtigen Schwefelverbindungen an dem Zahn 109 durch einen flexiblen Kanal 201 ab. Im Inneren des elektronischen Dentalgerätes 200 sind die fluoreszierenden Partikel 101 angeordnet, über die die abgesaugten Schwefelverbindungen geleitet werden.

Zusätzlich umfasst das elektronische Gerät 200 eine Beleuchtungsvorrichtung 203, die die fluoreszierenden Partikel 101 zur Fluoreszenz anregt. Das elektronische Dentalgerät 200 umfasst einen Lichtsensor 205 zum Erfassen einer Intensität des Fluoreszenzlichts, wie beispielsweise eine Leuchtdiode. Nimmt die Intensität des Fluoreszenzlichts ab, so liegt eine kariöse Veränderung an der Stelle vor, von wo die Schwefelverbindungen abgesaugt worden sind. Auf diese Weise können fluoreszierende Partikel 101 in der Analytik verwendet werden, um präzise Aussagen über kariöse Veränderungen von Zähnen zu erhalten.

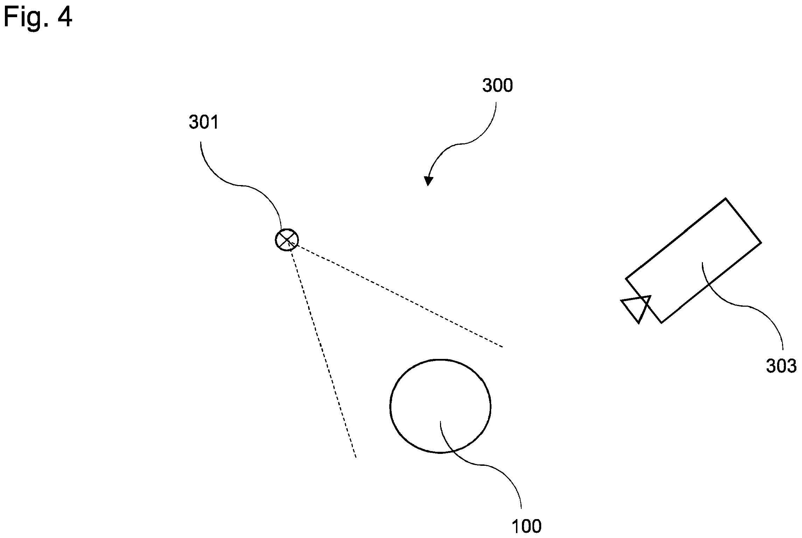

Fig. 4 zeigt eine schematische Ansicht eines Karieserfassungssystems 300. Das Karieserfassungssystem 200 umfasst die Karieserfassungsvorrichtung und eine zusätzliche Lichtquelle 301 zum Emittieren von Strahlung mit einer Wellenlänge, die kleiner als 500 nm ist. Die Lichtquelle 301 kann aktiviert werden, um die Fluoreszenz der Karieserfassungsvorrichtung anzuregen. Die Lichtquelle 301 ist beispielsweise durch eine Leuchtdiode gebildet. Das Licht der Lichtquelle 301 trifft auf die Karieserfassungsvorrichtung.

Zusätzlich umfasst das Karieserfassungssystem 300 einen Lichtsensor 303 zum Erfassen von Fluoreszenzlicht, das von Karieserfassungsvorrichtung mit den fluoreszierenden Partikeln 101 ausgesendet wird. Der Lichtsensor 303 kann durch eine elektronische Kamera gebildet sein, mit der das Fluoreszenzlicht der Karieserfassungsvorrichtung erfasst werden kann. Die elektronische Kamera erzeugt einen Datensatz, der in einer elektronischen Akte gespeichert werden kann. Eine Erfassung und Analyse der Fluoreszenzstrahlung kann jedoch auch mit einem Mobiltelefon durchgeführt werden, das mit einer elektronischen Kamera und einem Filter ausgestattet ist Die Karieserfassungsvorrichtung kann mittels unterschiedlicher Verfahren hergestellt werden. In einem ersten Verfahren kann die Karieserfassungsvorrichtung in einem dreidimensionalen Druckverfahren hergestellt werden. In dem dreidimensionalen Druckverfahren Fertigungsverfahre wird das herstellungsmaterial Schicht für Schicht aufgetragen, so dass dreidimensionale Gegenstände erzeugt werden können.

Während diesem dreidimensionalen Druckverfahren werden die fluoreszierende Partikel 101 eingebracht, die bei Kontakt mit der Schwefelverbindung die Fluoreszenzeigenschaft verlieren. Dies kann zum einen dadurch erfolgen, dass ein Herstellungsmaterial verwendet wird, in dem die fluoreszierenden Partikel 101 vor der Verarbeitung durch das Druckverfahren zugemischt worden sind. Zum anderen können die fluoreszierende Partikel 101 ein eigener Teil des Druckverfahrens sein und separat zum Herstellungsmaterial beim schichtweisen Aufbau zugeführt werden. Dadurch gelingt es die fluoreszierenden Partikel 101 an bestimmten Positionen der Karieserfassungsvorrichtung anzuordnen.

In einem zweiten Verfahren kann die Karieserfassungsvorrichtung durch Tiefziehen einer Folie mit fluoreszierenden Partikeln 101 hergestellt werden, die bei Kontakt mit einer Schwefelverbindung die Fluoreszenzeigenschaft verlieren. Beim Tiefziehen wird die Folie in einen einseitig offenen Hohlkörper umgeformt, indem dieses auf oder in einen Formkörper gezogen wird.

Die fluoreszierenden Partikel 101 sind homogen in der Folie verteilt. Allerdings ist es auch möglich eine Folie zu verwenden, bei der die fluoreszierenden Partikel 101 in einer oder mehreren Schichten angeordnet sind. Die fluoreszierenden Partikel 101 in der Folie können in bestimmten vorgegebenen Abständen angeordnet sein, beispielsweise in einer hexagonalen oder kubischen Struktur. Es kann auch eine Folie verwendet werden, die an unterschiedlichen Stellen eine unterschiedliche Dichte der fluoreszierenden Partikel 101 umfasst. So lassen sich bestimmte Bereiche der Karieserfassungsvorrichtung mit einer höheren oder niedrigeren Dichte an fluoreszierenden Partikeln 101 herstellen. Auf diese Weise können bestimmte Bereiche sensitiver auf kariöse Veränderungen reagieren. Beispielsweise kann auf diese Weise eine strukturierte Bissschiene erzeugt werden, um Sensitivität zu erhöhen.

In einem dritten Verfahren kann die Karieserfassungsvorrichtung durch Herausfräsen aus einem Block, wie beispielsweise einem Rohling, mit fluoreszierenden Partikeln 101 hergestellt werden, die bei Kontakt mit einer Schwefelverbindung die Fluoreszenzeigenschaft verlieren. Auch in dem Block können die fluoreszierenden Partikel 101 homogen verteilt sein. Allerdings können die fluoreszierenden Partikel 101 auch in einer oder mehreren Schichten oder räumlichen Bereichen des Blocks angeordnet sein. Es kann auch ein Block verwendet werden, der an unterschiedlichen Stellen eine unterschiedliche Dichte der fluoreszierenden Partikel 101 umfasst.

Anschließend kann innerhalb des Verfahrens Strahlung oder Licht mit einer Wellenlänge emittiert werden, die kleiner als 500 nm ist, vorzugsweise kleiner als 400 nm ist. Diese Strahlung trifft auf die Karieserfassungsvorrichtung und regt die fluoreszierenden Partikel 101 der Karieserfassungsvorrichtung zur Fluoreszenz an. Wenn diese zuvor mit einer kariösen Stelle in Kontakt gekommen sind, findet eine geringere Fluoreszenz statt, als wenn dies nicht der Fall gewesen ist.

Alle in Verbindung mit einzelnen Ausführungsformen der Erfindung erläuterten und gezeigten Merkmale können in unterschiedlicher Kombination in dem erfindungsgemäßen Gegenstand vorgesehen sein, um gleichzeitig deren vorteilhafte Wirkungen zu realisieren.

Alle Verfahrensschritte können durch Vorrichtungen implementiert werden, die zum Ausführen des jeweiligen Verfahrensschrittes geeignet sind. Alle Funktionen, die von gegenständlichen Merkmalen ausgeführt werden, können ein Verfahrensschritt eines Verfahrens sein.

Der Schutzbereich der vorliegenden Erfindung ist durch die Ansprüche gegeben und wird durch die in der Beschreibung erläuterten oder den Figuren gezeigten Merkmale nicht beschränkt.

### BEZUGSZECHENLISTE

- 100: Bissschiene
- 101: fluoreszierende Partikel
- 103: Quantenpunkte
- 105: räumlicher Bereich
- 107: kariöse Stelle
- 109: Zahn

- 200: elektronisches Gerät
- 201: Kanal
- 203: Beleuchtungsvorrichtung
- 205: Lichtsensor

- 300: Karieserfassungssystem
- 301: Lichtquelle
- 303: Lichtsensor

## Patentansprüche

1. Karieserfassungsvorrichtung (100, 200), mit:
- fluoreszierenden Partikeln (101), die bei Kontakt mit einer Schwefelverbindung die Fluoreszenzeigenschaft verlieren.

2. Karieserfassungsvorrichtung (100, 200) nach Anspruch 1, wobei die Schwefelverbindung Schwefelwasserstoff oder organische Schwefelverbindungen umfasst.

3. Karieserfassungsvorrichtung (100, 200) nach einem der vorangehenden Ansprüche, wobei die fluoreszierenden Partikel (101) Zinksulfid oder Cadmiumtellurid umfassen.

4. Karieserfassungsvorrichtung (100, 200) nach einem der vorangehenden Ansprüche, wobei die fluoreszierenden Partikel (101) durch Quantenpunkte (103) gebildet sind.

5. Karieserfassungsvorrichtung (100, 200) nach Anspruch 4, wobei die Quantenpunkte (103) eine vorgegebene Form und/oder vorgegebene Abmessung aufweisen.

6. Karieserfassungsvorrichtung (100, 200) nach einem der vorangehenden Ansprüche, wobei die fluoreszierenden Partikel (101) oder Quantenpunkte (103) homogen oder in einer vorgegebenen dreidimensionalen Struktur angeordnet sind.

7. Karieserfassungsvorrichtung (100, 200) nach einem der vorangehenden Ansprüche, wobei die fluoreszierenden Partikel (101) oder Quantenpunkte (103) in einer vorgegebenen Dichte angeordnet sind.

8. Karieserfassungsvorrichtung (100, 200) nach Anspruch 6, wobei die fluoreszierenden Partikel (101) in einem ersten räumlichen Bereich (105-1) der Karieserfassungsvorrichtung (100) in einer ersten Dichte angeordnet sind und einem zweiten räumlichen Bereich (105-2) der Karieserfassungsvorrichtung (100) in einer zweiten Dichte angeordnet sind.

9. Karieserfassungsvorrichtung (100, 200) nach einem der vorangehenden Ansprüche, wobei die Karieserfassungsvorrichtung (100) eine Zahnbürste, eine Zahnschiene, eine Aufbissschiene, eine Aligner-Schiene, eine Kunststofffolie, eine Zahnseide, ein Kaugummi, eine Teilprothese, ein Lack, ein Zement, oder ein Bracket oder ein elektronisches Gerät ist.

10. Karieserfassungssystem (300) mit einer Karieserfassungsvorrichtung (100) nach einem der Ansprüche 1 bis 9 und einer Lichtquelle (301) zum Emittieren von Strahlung mit einer Wellenlänge, die kleiner als 500 nm ist.

11. Karieserfassungssystem (300) nach Anspruch 11, wobei das Karieserfassungssystem (300) einen Lichtsensor (303) zum Erfassen von Fluoreszenzlicht umfasst.

12. Verfahren zum Herstellen einer Karieserfassungsvorrichtung (100), mit den Schritten:
- Herstellen der Karieserfassungsvorrichtung (100) in einem Formgebungsverfahren; und
- Einbringen von fluoreszierenden Partikeln (101) in die Karieserfassungsvorrichtung (100), die bei Kontakt mit einer Schwefelverbindung die Fluoreszenzeigenschaft verlieren.

13. Verfahren nach Anspruch 12, wobei die Karieserfassungsvorrichtung (100) in einem dreidimensionalen Druckverfahren hergestellt wird und die fluoreszierenden Partikel (101) durch das Druckverfahren in die Karieserfassungsvorrichtung (100) eingebracht werden.

14. Verfahren nach Anspruch 12, wobei die Karieserfassungsvorrichtung (100) durch Tiefziehen einer Folie hergestellt wird und die fluoreszierenden Partikel (101) durch die Folie in die Karieserfassungsvorrichtung (100) eingebracht werden.

15. Verfahren nach Anspruch 12, wobei die Karieserfassungsvorrichtung (100) durch Herausfräsen aus einem Rohling hergestellt wird und die fluoreszierenden Partikel (101) durch den Rohling in die Karieserfassungsvorrichtung (100) eingebracht werden.
